(19) Europäisches Patentamt
European Patent Office
Office européen des brevets

(11) Veröffentlichungsnummer: **0 299 201**
**A2**

# (12) EUROPÄISCHE PATENTANMELDUNG

(21) Anmeldenummer: **88109209.2**

(22) Anmeldetag: **09.06.88**

(51) Int. Cl.[4]: **A61K 31/13 , A61K 31/685 , A61K 31/215 , A61K 31/16 , A61K 31/70**

Die Bezeichnung der Erfindung wurde geändert (Richtlinien für die Prüfung im EPA, A-III, 7.3).

(30) Priorität: **12.06.87 DE 3719720**

(43) Veröffentlichungstag der Anmeldung: **18.01.89 Patentblatt 89/03**

(84) Benannte Vertragsstaaten: **CH DE FR GB IT LI**

(71) Anmelder: **Hoerrmann, Wilhelm, Dr.**
**Staltacherstrasse 34**
**D-8127 Iffeldorf(DE)**

(72) Erfinder: **Hoerrmann, Wilhelm, Dr.**
**Staltacherstrasse 34**
**D-8127 Iffeldorf(DE)**

(74) Vertreter: **Flosdorff, Jürgen**
**Alleestrasse 33**
**D-8100 Garmisch-Partenkirchen(DE)**

## (54) Fett-Aminoalkohol enthaltendes Arzneimittel.

(57) Diese Patentanmeldung betrifft die Verwendung von Fett-amino-alkoholen und Verbindungen davon als Arzneimittel bei AIDS und anderen Erkrankungen.

EP 0 299 201 A2

## Arzneimittel

Die Erfindung betrifft ein Arzneimittel, das als Wirksubstanz mindestens ein Isomer eines Fett-amino-alkohols oder dessen pnarmazeutisch annehmbaren Abkömmling gegebenenfalls zusammen mit üblichen Trägern und/oder Hilfsmitteln enthält.

Ein wichtiger solcher Fett-amino-alkohol ist das Sphingosin, wobei es sich um 2 - Amino - 4 octadecen 1,3 diol handelt.

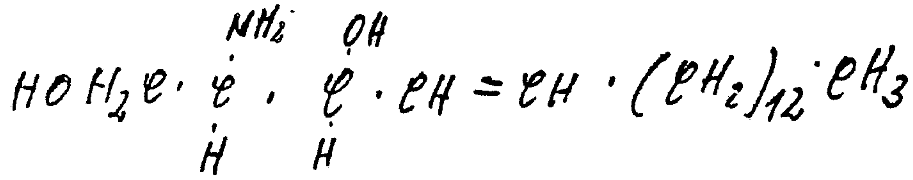

Es ist besonders zu beachten, daß diese Fett-amino-alkohole in zahlreichen isomeren Formen auftreten können. Da beim Sphingosin das Kohlenstoffatom Nr. 2 ebenso wie das Kohlenstoffatom Nr. 3 die Amino- bzw. die Hydroxylgruppe tragend, beide asymmetrisch sind, ergeben sich bereits daraus 4 optische Isomere. Sie kombinieren sich mit 2 geometrischen Isomeren auf Grund der Doppelbindung. Es gibt also insgesamt 8 Isomere des Sphingosins.

Eines dieser Isomeren ist das trans - d - erythro - 2 amino -4 octadecen 1,3 diol.

Die erfindungsgemäßen Arzneimittel können die Fett-amino-alkohole, so auch das Sphingosin, auch in Form von pharmazeutisch verträglichen Abkömmlingen enthalten. Dazu gehören insbesondere die Ether. Ester, Amide, Salze und Säureadditionssalze der genannten Verbindungen. Typische Beispiele hierfür sind die N-acyl-Derivate mit gesättigten oder ungesättigten Fettsäuren, auch als Ceramide bezeichnet. Zusammen mit Phosphorylcholin, Phosphorylserin, Phosphorylethanolamin und Phosphorylinositol handelt es sich um Sphingomyeline.

In Verbindung von Ceramid mit Zuckermolekülen (Glukose, Galaktose, N - acetyl - Glukosamin, N - acetyl - Galaktosamin, Mannose, Neuraminsäure und anderen) handelt es sich um Cerebroside, um Ganglioside, allgemein gesprochen um Glykolipide bzw. Glykosphingolipide. Die genannten Verbindungen können durch die zusätzliche Gabe von Phosphoglyceriden und Plasmalogenen in ihrer Wirkung unterstützt werden.

Die hier genannten Verbindungen sind bekannt und können nach an sich bekannten Verfahren hergestellt werden.

Das zentrale Molekül der Glyko-Sphingolipide und Sphingomyeline ist das Sphingosin, da es den jeweiligen molekularen Gesamtkomplex in der Zellmembran verankert, wobei es säureamidartig an eine langkettige oder ungesättigte Fettsäure gebunden ist. Störungen in der molekularen Strüktur des Sphingosins bzw. Ceramids haben deshalb schwerwiegende Folgen für die Zellmembran und ihre Anhangsgebilde.

Die Hauptanwendungsgebiete dieses Arzneimittels sind AIDS (das erworbene Immun-Defekt-Syndrom), Drogensucht, Haemophilie, und andere Erkrankungen, speziell des Nervensystems.

Der Anmelder dieser Patentanmeldung kam auf Grund seiner Studien zu dem Ergebnis, daß bei all diesen Erkrankungen, besonders aber bei AIDS Störungen in der Struktur und Funktion der köropereigenen Glyko-Sphingolipide und Sphingomyeline äußerst wesentlich sind. Eine besonders wesentliche Rolle kommt dabei dem Sphingosinbestandteil dieser Substanzen zu.

Störungen des Sphingosins und seiner Verbindungen stellen zumindest für einen Teil der Fälle eine biochemische Praedisposition für den Erwerb der genannten Krankheiten dar.

Mit den erfindungsgemäßen Arzneimitteln ist eine günstige Beeinflussung einer solchen praedispositionellen Vorschädigung ("Risikogruppen") ebenso möglich, wie die einer klinisch manifesten Erkrankung.

Die Isomere der Fett-amino-alkohole können per se aber auch in der Form ihrer Ceramide, Shingomyeline, Cerebroside oder Ganglioside zugeführt werden, wobei auch Bindungen an Proteine und Peptide möglich sind.

Chemisch sind die genannten Verbindungen bekannt und insofern können sie auch nach bekannten chemischen Verfahren hergestellt werden. Das trifft auch auf die Trennung der Isomeren zu.

Neu aber ist der Gebrauch dieser Substanzen bei Krankheiten wie AIDS, Drogensucht, Haemophilie und Erkrankungen des Nervensystems.

Nach allem unserem heutigen Wissen sind die genannten Verbindungen nicht toxisch und können deshalb in einem weiteren Dosierbereich angewandt werden. Ca. 100 - 400 mg/kg Körpergewicht gemessen am Fett-amino-alkohol können täglich gegeben werden.
Die Art der Zufuhr kann oral, anul und parenteral sein. Die Substanzen können in Pflanzenölen gelöst werden, in Kapseln oder Suppositorien eingeschlossen oder in Emulsionen gemischt werden. Ganz moderne Techniken, wie die Liposomen, können Verwendung finden.

## Ansprüche

1. Arzneimittel,
**gekennzeichnet durch**
einen Gehalt an einem Isomer eines Fett-amino-alkohols oder dessen pharmazeutisch annehmbaren Abkömmling als Wirkstoff, gegebenenfalls neben üblichen Trägerstoffen, Hilfsmitteln und/oder Zusatzstoffen.

2. Arzneimittel nach Anspruch 1,
dadurch gekennzeichnet, daß der Fett-amino-alkohol ein Sphingosin ist.

3. Arzneimittel nach Anspruch 2,
dadurch gekennzeichnet, daß das Isomer das trans-d-erythro-2 amino-4 octadecen 1,3 diol ist.

4. Arzneimittel nach einem der Ansprüche 1 bis 3,
dadurch gekennzeichnet, daß der Wirkstoff in Form von Ceramid, Sphingomyelin oder eines Glykolipids bzw. Glykosphingolipids vorliegt.

5. Arzneimittel nach einem der Ansprüche 1 bis 4,
dadurch gekennzeichnet, daß die genannten Verbindungen zusätzlich in Form von Peptid-Eiweißbindungen vorliegen.